(19) Europäisches Patentamt
European Patent Office
Office européen des brevets

(11) Veröffentlichungsnummer: **0 173 172 A2**

# (12) EUROPÄISCHE PATENTANMELDUNG

(21) Anmeldenummer: **85110230.1**

(22) Anmeldetag: **15.08.85**

(51) Int. Cl.[4]: **C 07 D 213/30**, C 07 D 213/50, C 07 D 409/10, C 07 D 213/55, C 07 D 213/48, C 07 D 213/80, C 07 D 405/10, A 61 K 31/44

(30) Priorität: **23.08.84 DE 3431004**

(43) Veröffentlichungstag der Anmeldung: **05.03.86 Patentblatt 86/10**

(84) Benannte Vertragsstaaten: **AT BE CH DE FR GB IT LI LU NL SE**

(71) Anmelder: **HOECHST AKTIENGESELLSCHAFT, Postfach 80 03 20, D-6230 Frankfurt am Main 80 (DE)**

(72) Erfinder: **Wess, Günther, Dr., Hainstrasse 35, D-6455 Erlensee (DE)**
Erfinder: **Bartmann, Wilhelm, Dr., Am Dachsbau 5, D-6232 Bad Soden am Taunus (DE)**
Erfinder: **Beck, Gerhard, Dr., Gustav-Freytag-Strasse 24, D-6000 Frankfurt am Main 1 (DE)**
Erfinder: **Lau, Hans-Hermann, Dr., Kastanienhain 29, D-6232 Bad Soden am Taunus (DE)**

(54) **Neue 3-Pyridylverbindungen und Verfahren zu ihrer Herstellung.**

(57) Die vorliegende Erfindung betrifft neue, in 3-Stellung substituierte Pyridine der Formel

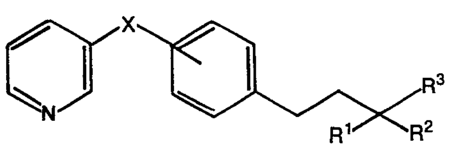

sowie ein Verfahren zu ihrer Herstellung. Die Verbindungen bewirken eine spezifische Hemmung der Thromboxan-Synthetase und können daher als Arzneimittel verwendet werden.

EP 0 173 172 A2

Neue 3-Pyridylverbindungen und Verfahren zu ihrer Herstellung

Pyridin und bestimmte Derivate sind Hemmer der Thromboxan-Synthetase (Miyamoto, T. Taniguchi, K., Tanouchi.T., and Hirata, F., Adv. Prostaglandin Thromboxane Res. 6, 443, (1980)).

Das Enzym Thromboxan-Synthetase katalysiert innerhalb des Arachidonsäuremetabolismus die Umwandlung der Prostaglandinendoperoxide ($PGH_2$ bzw. $PGG_2$) in Thromboxan $A_2$ ($TXA_2$). $TXA_2$ ist biologisch hoch aktiv: es induziert die Aggregation der Blutplättchen und wirkt außerdem auf die glatte Muskulatur stark konstriktiv. Es spielt eine wesentliche Rolle bei der Haemostase, bei pathologischen Situationen mit erhöhter Tendenz zu Gefäßkrämpfen und/oder Thrombose. Ferner wirkt $TXA_2$ in vitro und in vivo stark kontrahierend auf die Bronchialmuskulatur (B. Samuelsson, Angew. Chemie 95, 854 (1983).

Die neuen 3-Pyridylverbindungen, die in der vorliegenden Erfindung beschrieben werden zeichnen sich durch eine spezifische Hemmwirkung auf die Thromboxan-Synthetase aus.

Sie eignen sich daher zur Prophylaxe oder Behandlung von Krankheiten mit gestörter (erhöhter) Thrombocytenaggregationsneigung, sowie bei pathologisch erhöhten Thromboxanspiegeln, wie sie bei Ischämie, Angina pectoris, thromboembolischen Erkrankungen, Atherosklerose, Koronarkrämpfen, Arrhythmien, cerebralen ischämischen Anfällen, Migräne und anderen vaskulären Kopfschmerzen,

Myokardinfarkt, Hypertension, Atmungsstörungen wie Asthma und Apnoe, Entzündungskrankheiten sowie mikrovaskulären Komplikationen bei diabetes mellitus gefunden werden. Die erfindungsgemäßen Verbindungen beeinflussen günstig Erkrankungen mit erhöhten Thromboxanspiegeln in verschiedenen Organen, beispielsweise im Bereich der Nieren oder im Magen-Darm Bereich bei Colitis oder bei "inflammatory bowel disease". Außerdem sind die Verbindungen geeignet, die Proliferation von Tumorzellen zu verlangsamen bzw. zu verhindern. Einige der erfindungsgemäßen Verbindungen besitzen außerdem ausgeprägte hypolipidämische Wirkungen mit bevorzugter Verminderung der atherogenen LDL und VLDL Lipoproteinfraktionen. Sie eignen sich daher als Lipidsenker und zur Behandlung der Arteriosklerose. Gegenstand der vorliegenden Erfindung sind neue in Position 3 substituierte Pyridine der Formel I, die eine spezifische Hemmung der Thromboxan-Synthetase bewirken.

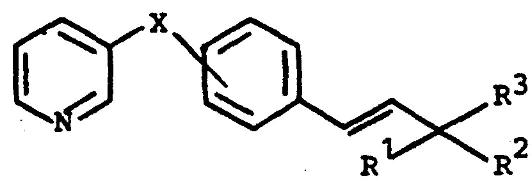

I

o, m, p

In der allgemeinen Formel I bedeuten:

X eine Vinylengruppe oder Ethylengruppe,
$R^1$ und $R^2$ zusammen die Carbonylgruppe, oder $R^1$ Wasserstoff und $R^2$ der Rest $-OR^4$, worin $R^4$ Wasserstoff darstellt oder

a) einen verzweigten oder unverzweigten aliphatischen Acylrest mit bis zu 10 C-Atomen,

b) den Benzylcarbonyl oder Benzoylrest, wobei der Phenylrest unsubstituiert oder 1- bis 3-fach substituiert ist mit Halogen oder $C_1$-$C_4$-Alkyl,

c) den Rest

d) verzweigtes oder unverzweigtes Alkyl mit 1-10 C-Atomen,

e) den Benzylrest, wobei der Phenylkern unsubstituiert oder 1- bis 3-fach substituiert ist mit Halogen oder $C_1$-$C_4$-Alkyl, oder

f) den Rest ,

$R^3$ einen Phenylrest, der im Kern 1- bis 3-fach substituiert sein kann mit Halogen, Trifluormethyl, und/oder Alkyl oder Alkoxy mit 1-6 C-Atomen, oder einen cycloaliphatischen Rest mit 3-8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3-8 Kohlenstoffatomen, wobei die aliphatischen Reste ihrerseits substituiert sein können mit

a) einem geradkettigen oder verzweigten Alkoxyrest mit bis zu 10 Kohlenstoffatomen oder einem Cycloalkoxyrest mit bis zu 6 Kohlenstoffatomen oder einem geradkettigen oder verzweigten Alkenyloxy- oder Alkinyloxyrest mit 3 bis 6 Kohlenstoffatomen,

b) Halogen, Cycloalkyl mit 3-7 C-Atomen, einem unsubstituierten Phenyl-, α- oder ß-Thienyl oder α- oder ß-Furylrest oder einem Phenyl-, Thienyl- oder Furylrest, welche ihrerseits im Kern 1- bis 3-fach substituiert sind mit Halogen, Trifluormethyl und/oder Alkyl oder Alkoxy mit 1-6 C-Atomen,

c) einem unsubstituierten Phenoxy-, α- oder ß-Thienyloxy- oder Cycloalkoxyrest mit 3-7 Kohlenstoffatomen oder einem der genannten Reste, welche seinerseits im Kern 1- bis 3-fach substituiert ist mit Halogen, Trifluormethyl und/oder Alkyl oder Alkoxy mit je 1-6 C-Atomen,

d) einem Heteroarylrest.

Die Substituenten haben bevorzugt die folgende Bedeutung:

$R^1$ und $R^2$ zusammen die Carbonylgruppe, oder $R^1$ Wasserstoff und $R^2$ der Rest $-OR^4$, worin $R^4$ Wasserstoff darstellt oder

a) verzweigtes oder unverzweigtes Alkanoyl mit bis zu 6 C-Atomen,

b) den Benzylcarbonyl oder Benzoylrest, wobei der Phenylrest unsubstituiert oder einfach substituiert ist mit Fluor, Chlor oder Methyl,

c) den Rest 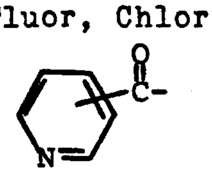

d) verzweigtes oder unverzweigtes Alkyl mit 1-6 C-Atomen,

e) den Benzylrest, wobei der Phenylkern unsubstituiert oder mehrfach substituiert ist mit Chlor, Fluor oder Methyl, oder

f) den Rest 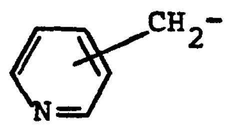

$R^3$ einen cycloaliphatischen Rest mit 3-8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3 bis 8 Kohlenstoffatomen, wobei die aliphatischen Reste ihrerseits substituiert sein können mit

a) einem geradkettigen oder verzweigten Alkoxyrest mit bis zu 6 Kohlenstoffatomen oder einem Cycloalkoxyrest mit bis zu 6 Kohlenstoffatomen oder einem geradkettigen oder verzweigten Alkylenoxy- oder Alkinyloxyrest mit 3 bis 6 Kohlenstoffatomen,

b) Cycloalkyl mit 3-7 C-Atomen, einem unsubstituierten Phenyl-, α- oder β-Thienyl oder α- oder β-Furylrest oder einem Phenyl-, Thienyl- oder Furylrest, welche ihrerseits im Kern 1- bis 3-fach substituiert sind mit Halogen, Trifluormethyl und/oder Alkyl oder Alkoxy mit 1-6 C-Atomen,

c) einem unsubstituierten Phenoxy-, α- oder β-Thienyloxy- oder Cycloalkoxyrest mit 3-7 Kohlenstoffatomen oder einem der genannten Reste, welche seinerseits im Kern 1- bis 3-fach substituiert ist mit Halogen, Trifluoromethyl und/oder Alkyl oder Alkoxy mit je 1-4 C-Atomen,

d) einem Heteroarylrest.

Besonders bevorzugt sind die folgenden Substituenten:

$R^1$ und $R^2$ zusammen die Carbonylgruppe, oder $R^1$ Wasserstoff und $R^2$ den Rest $-OR^4$ worin $R^4$ Wasserstoff, Benzyl, Alkyl mit 1-6 C-Atomen oder den Rest 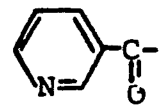

oder 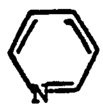 $-CH_2-$darstellt,

$R^3$ einen cycloaliphatischen Rest mit 3-8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3 bis 8 Kohlenstoffatomen, wobei die aliphatischen Reste ihrerseits substituiert sein können mit

a) einem geradkettigen oder verzweigten Alkoxyrest mit bis zu 10 Kohlenstoffatomen,

b) Cycloalkyl mit 3-7 C-Atomen, einem unsubstituierten Phenyl-, α- oder β-Thienyl oder α- oder β-Furylrest oder einem Phenyl-, Thienyl- oder Furylrest, welche ihrerseits im Kern 1- bis 3-fach substituiert sind mit Halogen, Trifluormethyl und/oder Alkyl oder Alkoxy mit 1-6 C-Atomen,

c) einem unsubstituierten Phenoxy-, α- oder β-Thienyloxy- oder Cycloalkoxyrest mit 3-7 Kohlenstoffatomen,

d) einem 1-Imidazolylrest.

insbesondere die Reste:

n-Pentyl, 1,1-Dimethylpentyl, Cyclopentylmethyl, Cyclohexylmethyl, Methoxymethyl, Ethoxymethyl, Propoxymethyl, Butoxymethyl, Pentoxymethyl, Hexyloxymethyl, Heptyloxymethyl, Cyclopentyloxymethyl, Cyclohexyloxymethyl, Dimethylpentoxymethyl, 1,1-Dimethyl-2-ethoxyethyl, Phenoxymethyl, 2-Ethoxyethyl, 2-Butoxyethyl, 3-Ethoxypropyl, 5-Methoxypentyl, (2-Ethoxy)ethoxymethyl, 3-Chlorphenoxymethyl, 1,1-Dimethyl-2-benzyloxyethyl, 3-Methoxycarbonylpropyl, 4-Methoxycarbonylbutyl, 5-Methoxycarbonylpentyl, 3-Carboxypropyl, 4-Carboxybutyl, 5-Carboxypentyl, 4-(3-Chlorbenzyloxy)phenyl, Phenylethoxymethyl, 3-Thienyloxymethyl, 2-Thienyloxymethyl, 2-(1-Imidazolyl)ethyl, Dimethyl-(1-imidazolyl)

Die Erfindung umfaßt ferner Säureadditionssalze mit anorganischen oder organischen Säuren z.B. Chlorwasserstoffsäure, Bromwasserstoffsäure, Schwefelsäure, Phosphorsäure oder Salpetersäure, Essigsäure, Propionsäure, Oxalsäure, Malonsäure, Glykolsäure, Bernsteinsäure, Maleinsäure, Fumarsäure, Äpfelsäure, Weinsäure, Zitronensäure, Benzoesäure oder Zimtsäure.

Gegenstand der Erfindung ist ferner ein Verfahren zur Herstellung der Verbindungen der allgemeinen Formel I, dadurch gekennzeichnet, daß man

a) die entsprechenden ortho-, meta- und parasubstituierten Benzaldehyde der Formel II

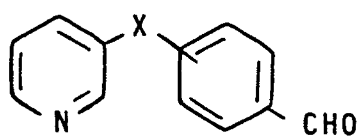

II

o, m, p

nach Horner-Emmons-Wittig mit einem Phosphonsäureester der allgemeinen Formel III,

$$(R^5O)_2\underset{\underset{O}{\|}}{P}-CH_2-\underset{\underset{O}{\|}}{C}-R^3 \qquad III$$

wobei $R^3$ die in der allgemeinen Formel I angegebene Bedeutung besitzt und $R^5$ $C_1$-$C_4$-Alkyl, vorzugsweise Methyl und Ethyl bedeutet, zu Verbindungen der allgemeinen Formel IV umsetzt,

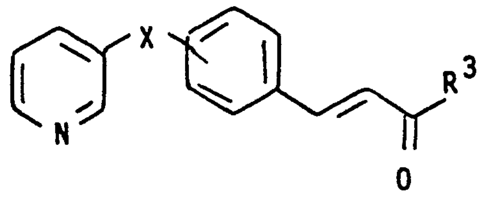

IV

o, m, p

wobei $R^3$ die zur Formel I angegebene Bedeutung hat,

b) gegebenenfalls die Enone der allgemeinen Formel IV mit einem Reduktionsmittel zu den Alkoholen der allgemeinen Formel V reduziert,

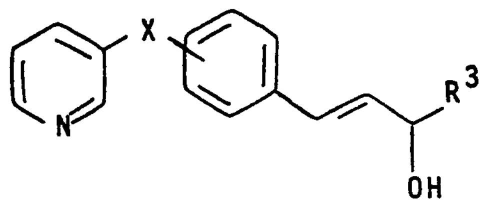

V

o, m, p

worin $R^3$ die zur Formel I gegebene Bedeutung hat,

c) gegebenenfalls die Alkohole der Formel V in Form ihrer Racemate oder als reine Enantiomere mit einem reaktionsfähigen Derivat einer Carbonsäure zu den Estern der allgemeinen Formel VI umsetzt,

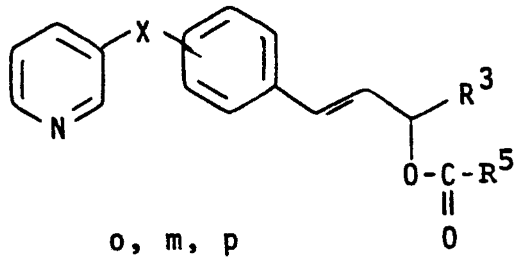

VI

o, m, p

worin $R^3$ die zur Formel I angegebene Bedeutung hat und $R^4$ einen verzweigten oder unverzweigten aliphatischen Acylrest mit bis zu 10 C-Atomen, oder den Benzylcarbonyl- oder Benzoylrest, worin der Phenylkern 1- bis 3-fach mit Halogen oder $C_1$-$C_4$-Alkyl substituiert sein kann, oder den Rest 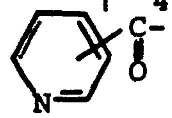 bedeutet, oder

d) gegebenenfalls die Alkohole der Formel V in Form ihrer Racemate oder als reine Enantiomere mit einem entsprechenden Halogenid, Tosylat oder Mesylat zu den Ethern der allgemeinen Formel VII umsetzt,

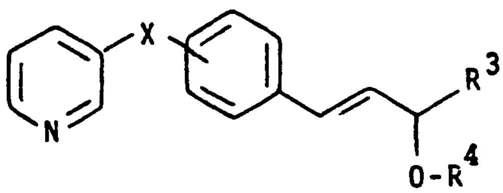

VII

o, m, p

worin $R^4$ $C_1$-$C_{10}$-Alkyl, Benzyl, worin der Phenylkern 1- bis 3-fach mit Halogen oder $C_1$-$C_6$-Alkyl substituiert sein kann, oder den Rest 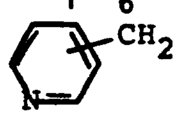 darstellt.

Zur Herstellung der im erfindungsgemäßen Verfahren als Ausgangsmaterial verwendeten 2-(3-Pyridyl)ethenylbenzaldehyde geht man von den entsprechenden o-, m- bzw. p-Chlormethylbenzaldehyden aus. Die Chlormethylbenzaldehyde können in Analogie zu bekannten Verfahren (z.B. R. Grice, L.N. Owen, J. Chem. Soc. 1963, 1947 oder I.W. Baker, I.A.L. Brieux, D.G. Saunders, J. Chem. Soc. 1956, 404) hergestellt werden.

Die Ethylenacetale letzterer Aldehyde werden mit Phosphorigsäuretriethylester in bekannter Weise (B.A.Arbuzov,

Pure Appl. Chem. 9, 307 (1964)) in die entsprechenden Phosphonate überführt. Diese wiederum werden in bekannter Weise (W.S. Wadsworth et al. J.Am.Chem.Soc. 83, 1733 (1961), J.Org.Chem. 30, 680 (1965)) mit Pyridin-3-aldehyd zu den Ethylenacetalen der entsprechenden o-, m- bzw. p-[2-(3-Pyridyl)ethenyl]benzaldehyden umgesetzt. Letztere können katalytisch zu den o-, m- bzw. p-[2-(3-Pyridyl) ethyl]benzaldehydethylenacetalen hydriert werden.

Die o-, m-, p-substituierten Benzaldehyde der Formel II werden nach Horner-Emmons-Wittig mit einem Phosphonsäureester der allgemeinen Formel III umgesetzt, wobei eine bevorzugte Ausführungsform darin besteht, daß man den Phosphonsäureester der Formel III mit dem Aldehyd der Formel II in Dimethoxyethan unter Verwendung von DBU (1,8-Diazabicyclo[5.4.0]undec-7-en) als Base bei Raumtemperatur bis Rückflußtemperatur 1-36 Stunden reagieren läßt.

Die Phosphonsäureester der Formel III könnnen nach literaturbekannten Verfahren (siehe z.B. J.Am.Chem. Soc. 88, 5654 (1966)) hergestellt werden.

Die Alkohole der allgemeinen Formel V erhält man in Form ihrer Racemate, wenn man ein Enon der allgemeinen Formel IV mit einem komplexen Metallhydrid, vorzugsweise mit einem Alkaliboranat, vorzugsweise zwischen -10 °C und Raumtemperatur in Methanol, Ethanol oder Ethern wie DME, THF ggf. unter Wasserzusatz reduziert.

Die Acylierung der Alkohole der Formel V zu den Estern der Formel VI erfolgt in der allgemein üblichen Weise mit Acylhalogeniden (wie z.B. Nicotinsäurechlorid) oder mit Säureanhydriden in Gegenwart von Basen wie Pyridin, Triethylamin u.a..

Die Veretherung der Alkohole der Formel V zu den Ethern der Formel VII erfolgt in der allgemein üblichen Weise mit Halogeniden (wie z.B. Benzylhalogenid), Mesylaten oder Tosylaten in Gegenwart von Basen wie z.B. Natriumhydrid in geeigneten Lösungsmitteln wie z.B. DMF.

Sofern die einzelnen Reaktionsprodukte nicht bereits in genügend reiner Form anfallen, so daß sie für den folgenden Reaktionsschritt eingesetzt werden können, empfiehlt sich eine Reinigung mittels Kristallisation oder Säulen-, Dünnschicht- oder Hochdruckflüssigkeitschromatographie.

Die Säureadditionssalze von Verbindungen der allgemeinen Formel I werden durch Zugabe der entsprechenden anorganischen oder organischen Säuren meistens in Form einer Lösung zu Lösungen der entsprechenden Imidazolverbindung der allgemeinen Formel I erhalten. Als Lösungsmittel dienen z.B. Wasser, Alkohole aber auch verschiedene Ether und Ester.

Außer den in den Beispielen beschriebenen Verbindungen lassen sich nach den erfindungsgemäßen Verfahren die folgenden Verbindungen herstellen:

E,E-4-Ethoxy-1-[4-(2-(3-pyridyl)ethenyl)phenyl]but-1-en-3-ol
E,E-5-Ethoxy-1-[4-(2-(3-pyridyl)ethenyl)phenyl]pent-1-en-3-ol
E,E-5-Butoxy-1-[4-(2-(3-pyridyl)ethenyl)phenyl]pent-1-en-3-ol
E,E-4-Ethoxy-1-[4-(2-(3-pyridyl)ethenyl)phenyl]but-1-en-3-on
E,E-5-Ethoxy-1-[4-(2-(3-pyridyl)ethenyl)phenyl]pent-1-en-3-on
E,E-5-Butoxy-1-[4-(2-(3-pyridyl)ethenyl)phenyl]pent-1-en-3-on
E,E-6-Ethoxy-1-[4-(2-(3-pyridyl)ethenyl)phenyl]hex-1-en-3-on
E,E-6-Ethoxy-1-[4-(2-(3-pyridyl)ethenyl)phenyl]hex-1-en-3-ol
E,E-7-Methoxy-1-[4-(2-(3-pyridyl)ethenyl)phenyl]hept-1-en-3-on

E,E-7-Methoxy-1-[4-(2-(3-pyridyl)ethenyl)phenyl]hept-1-en-3-ol
E,E-8-Methoxy-1-[4-(2-(3-pyridyl)ethenyl)phenyl]oct-1-en-3-on
E,E-8-Methoxy-1-[4-(2-(3-pyridyl)ethenyl)phenyl]oct-1-en-3-ol
E,E-5-(1-Imidazolyl)-1-[4-(2-(3-pyridyl)ethenyl)phenyl]pent-1-en-3-ol
E,E-4,4-Dimethyl-4-(1-imidazolyl-1-[4-(2-(3-pyridyl)ethenyl)phenyl]but-1-en-3-on
E,E-4,4-dimethyl-4-(1-imidazolyl-1-[4-(2-(3-pyridyl)ethenyl)phenyl]but-1-en-3-ol
E,E-3-(3-Chlorphenyl)-1-[4-(2-(3-pyridyl)ethenyl)phenyl]prop-1-en-3-on
E,E-3-(3-Chlorphenyl)-1-[4-(2-(3-pyridyl)ethenyl)phenyl]prop-1-en-3-ol
E,E-4-Ethoxy-1-[4-(2-(3-pyridyl)ethyl)phenyl]but-1-en-3-ol
E,E-5-Ethoxy-1-[4-(2-(3-pyridyl)ethyl)phenyl]pent-1-en-3-ol
E,E-5-Butoxy-1-[4-(2-(3-pyridyl)ethyl)phenyl]pent-1-en-3-ol
E,E-4-Ethoxy-1-[4-(2-(3-pyridyl)ethyl)phenyl]but-1-en-3-on
E,E-5-Ethoxy-1-[4-(2-(3-pyridyl)ethyl)phenyl]pent-1-en-3-on
E,E-5-Butoxy-1-[4-(2-(3-pyridyl)ethyl)phenyl]pent-1-en-3-on
E,E-6-Ethoxy-1-[4-(2-(3-pyridyl)ethyl)phenyl]hex-1-en-3-on
E,E-6-Ethoxy-1-[4-(2-(3-pyridyl)ethyl)phenyl]hex-1-en-3-ol
E,E-7-Methoxy-1-[4-(2-(3-pyridyl)ethyl)phenyl]hept-1-en-3-on
E,E-7-Methoxy-1-[4-(2-(3-pyridyl)ethyl)phenyl]hept-1-en-3-ol
E,E-8-Methoxy-1-[4-(2-(3-pyridyl)ethyl)phenyl]oct-1-en-3-on
E,E-8-Methoxy-1-[4-(2-(3-pyridyl)ethyl)phenyl]oct-1-en-3-ol
E,E-5-(1-Imidazolyl)-1-[4-(2-(3-pyridyl)ethyl)phenyl]pent-1-en-3-ol
E,E-4,4-Dimethyl-4-(1-imidazolyl-1-[4-(2-(3-pyridyl)ethyl)phenyl]but-1-en-3-on
E,E-4,4-Dimethyl-4-(1-imidazolyl-1-[4-(2-(3-pyridyl)ethyl)phenyl]but-1-en-3-ol
E,E-3-(3-Chlorphenyl)-1-[4-(2-(3-pyridyl)ethyl)phenyl]prop-1-en-3-on
E,E-3-(3-Chlorphenyl)-1-[4-(2-(3-pyridyl)ethyl)phenyl]prop-1-en-3-ol

sowie die Nicotinsäureester, benzylether und /̄(3-Pyridyl)-methyl_7ether der hier und in Tabelle 2 aufgeführten Alkohole.

Die Verbindungen der Formel I zeichnen sich durch eine spezifische Hemmwirkung auf die Thromboxan-Synthetase aus und können daher als Arzneimittel zur Prophylaxe oder Behandlung von Krankheiten mit gestörter (erhöhter) Thrombocytenaggregationsneigung, sowie bei pathologisch erhöhten Thromboxanspiegeln, wie sie bei Ischämie, Angina pectoris, thrombo-embolischen Erkrankungen, Atherosklerose, Koronarkrämpfen, Arrhythmien, cerebralen ischämischen Anfällen, Migräne und anderen vaskulären Kopfschmerzen, Myokardinfarkt, Hypertension, Atmungsstörungen wie Asthma und Apnoe, Entzündungskrankheiten sowie mikrovaskulären Komplikationen bei Diabetes mellitus gefunden werden. Die erfindungsgemäßen Verbindungen beeinflussen günstig Erkrankungen mit erhöhten Thromboxanspiegeln in verschiedenen Organen, beispielsweise im Bereich der Nieren oder im Magen-Darm-Bereich bei Colitis oder bei "inflammatory bowel disease". Außerdem sind die Verbindungen geeignet, die Proliferation von Tumorzellen zu verlangsamen bzw. zu verhindern.

Einige der erfindungsgemäßen Verbindungen besitzen außerdem ausgeprägte hypolipidämische Wirkungen mit bevorzugter Verminderung der atherogenen LDL und VLDL Lipoproteinfraktionen. Sie eignen sich daher als Lipidsenker und zur Behandlung der Arteriosklerose. Die Verbindungen sind wirksam in Dosen von 0,01 mg/kg bis 10 mg/kg. Die verabreichte Einzeldosis kann zwischen 1 mg bis 500 mg liegen, die bevorzugte Tagesdosis zwischen 1 mg und 1 g bei oraler Applikation.

Arachidonsäuremetabolite sind an einer Vielzahl physiologischer und pathophysiologischer Prozesse beteiligt.

Bei der Regulation des Tonus der Blutgefäße und der Blutplättchenaggregation spielen Prostacyclin ($PGI_2$) und Thromboxan $A_2$ ($TXA_2$) eine wesentliche Rolle. Prostacyclin, das bevorzugt in den Endothelzellen der Blutgefäße aus Prostaglandinendoperoxid $H_2$ ($PGH_2$) entsteht, bewirkt eine Vasodilatation und verhindert die Aggregation der Blutplättchen. Die Umwandlung von Prostaglandinendoperoxid in Prostacyclin wird durch das Enzym Prostacyclin-Synthetase katalysiert. Thromboxan $A_2$ ist der physiologische Gegenspieler des Prostacyclins. Es entsteht vornehmlich in den Blutplättchen aus $PGH_2$. Das Enzym Thromboxan-Synthetase katalysiert diese Reaktion. $TXA_2$ bewirkt eine Aggregation der Blutplättchen und führt zu einer Vasokonstriktion. Soweit bekannt, ist Thromboxan der potenteste Vasokonstriktor im menschlichen Organismus (A.G. Hermann, P.M. Vonhoutte, H. Denolin, A. Goossens, Cardiovascular Pharmacology of the Prostaglandins, Raven Press, New York 1982). Ungleichgewichte zwischen Prostacyclin und Thromboxan $A_2$ führen zu pathophysiologischen Situationen. Eine Verschiebung des Gleichgewichtes zugunsten des Thromboxans führt daher zu einer Blättchenaggregation und Gefäßspasmen, sowie einer erhöhten Anfälligkeit gegenüber Atherothrombose (Lancet 1977, 479; Science 1976, 1135; Amer. J. Cardiology 41, 787 (1978); Lancet 1977, 1216;). Bei der experimentellen Atherosklerose ist die $PGI_2$-Bildung unterdrückt und die $TXA_2$-Bildung erhöht (Prostaglandins 14, 1025 und 1035 (1977)). Daher wird Thromboxan $A_2$ mit verschiedenen Anginen, myocardialer Infarktbildung, plötzlichem Herztod und Schlaganfällen in Verbindung gebracht (Thromb. Haemostasis 38, 132 (1977); Platelets, Prostaglandins and Cardiovascular System, Florenz, Februar 1984).

Ein anderes Gebiet, auf welchem ein Ungleichgewicht von $PGI_2$ / $TXA_2$ als ein beitragender Faktor angesehen wird, ist dasjenige von Migräne. Migränekopfschmerz ist mit Veränderungen der intra- und extra-cerebralen Blutströmung verbunden, insbesondere mit einer vor dem Auftreten des Kopfschmerzes erfolgenden Reduzierung der cerebralen Blutströmung und anschließenden Dilatation in beiden Gefäßbereichen während der Kopfschmerzphase. Blutplättchen von Migränepatienten besitzen eine größere Neigung zur Aggregation als diejenigen von normalen Individuen (J. clin. Pathol. 24, 250 (1971); J. Headache, 17, 101 (1977); Lancet 1978, 501).

Bei Patienten mit diabetes mellitus wird ein Ungleichgewicht zwischen Prostacyclin und Thromboxan $A_2$ als verantwortlich für die microvasculären Komplikationen angesehen. Plättchen von Diabetespatienten bilden erhöhte Mengen an $TXB_2$ und Malondialdehyd, siehe Symposium "Diabetes and Thrombosis-Implications for Therapy", Leeds, Großbritannien (April 1979). Weiterhin wurde gezeigt, daß bei Ratten mit experimentell erzeugter Diabetes die vaskuläre Prostacyclinbildung gehemmt wird und die $TXA_2$-Synthese aus den Plättchen erhöht ist, siehe IV. International Prostaglandin Conference, Washington, D.C. (Mai 1979).

Nichtsteroidale Antiinflammatorika hemmen die Cyclooxygenase, die die Umwandlung von Arachidonsäure in $PGH_2$ über $PGG_2$ katalysiert. Sie greifen daher in die Biosynthese sowohl des Thromboxans als aber auch in die Biosyntheses des Prostacyclins ein. Wertvoller wäre daher eine Verbindung, die spezifisch die Bildung von $TXA_2$ durch Blockade der $TXA_2$-Synthetase hemmt und den Prostacyclinweg unbeeinflußt läßt.

Die Verbindungen der Formel I eignen sich daher zur Vorbeugung oder Behandlung der oben aufgeführten Erkrankungen, die auf eine Hemmung der Thromboxansynthetase ansprechen.

Die Verbindungen der Formel I werden in verschiedenen Dosierformen verabreicht, z.B. oral in Form von Tabletten, Kapseln oder Flüssigkeiten, rektal in Form von Suppositorien, parenteral, subkutan oder intramuskulär, wobei intravenöse Verabreichung in Notsituationen bevorzugt wird.

Die erfindungsgemäßen Verbindungen der Formel I können als freie Basen oder in Form ihrer physiologisch unbedenklichen anorganischen oder organischen Säureadditionssalze zur Anwendung kommen. Die freien Basen und Säureadditionssalze können in Form ihrer wässrigen Lösungen oder Suspensionen oder auch gelöst oder suspendiert in pharmakologisch unbedenklichen organischen Lösungsmitteln wie ein- oder mehrwertigen Alkoholen wie z.B. Ethanol, Ethylenglykol oder Glycerin, in Triacetin, in Alkohol-Acetaldehyd-diacetalgemischen, Ölen wie z.B. Sonnenblumenöl oder Lebertran, Ethern wie z.B. Diethylenglykoldimethylether oder auch Polyethern wie z.B. Polyethylenglykol oder auch in Gegenwart anderer pharmakologisch unbedenklicher Polymerträger wie z.B. Polyvinylpyrrolidon zur Anwendung gelangen.

Als Zubereitungen können die üblichen galenischen Infusions- oder Injektionslösungen und Tabletten sowie örtlich anwendbare Zubereitungen wie Cremes, Emulsionen, Suppositorien oder Aerosole in Frage kommen.

Beispiel 1a

E-4-[2-(3-Pyridyl)ethenyl]benzaldehydethylenacetal

10.11 g (50.9 mmol) 4-Chlormethylbenzaldehydethylenacetal und 8.46 g (50.9 mmol) Triethylphosphit wurden ohne Lösungsmittel unter magnetischem Rühren bis zum Ende der Gasentwicklung (ca. 1h) auf 200-210 °C Ölbadtemperatur erhitzt. Nach dem Erkalten wurden 70 ml Dimethylformamid (über bas. Aluminiumoxid, Akt. I, filtriert) und 5.5 g (102.0 mmol) Natriummethylat zugegeben. Man kühlte auf 5 °C und tropfte unter magnetischem Rühren und Kühlen innerhalb 15 min eine Lösung von 5.45 g (50.9 mmol) Pyridin-3-aldehyd in 20 ml Dimethylformamid zu (wobei die Temp. auf 25 °C stieg) und ließ 1 h bei Raumtemp. nachrühren. Das Reaktionsgemisch wurde mit 250 ml Toluol verdünnt und zweimal mit Wasser gewaschen. Die vereinigten Waschphasen wurden zweimal mit Toluol extrahiert. Trocknen der vereinigten organischen Phasen über Magnesiumsulfat und Abziehen des Lösungsmittels lieferte 10.94 g eines gelblichen Feststoffes. Umkristallisation aus Cyclohexan ergab 8.74 g, Aufarbeitung der Mutterlauge weitere 0.60 g.
Ausbeute 9.34 g (36.9 mmol, 72 %)
Schmp.: 117-119 °C
$^{1}$H-NMR (60 MHz, $CDCl_3$): $\delta$ = 4,0 (m, 4H, Methylen-H), 5,8 (s, 1H, Methin-H), 7,0-8,8 (m, 10H olefinische und aromatische H)

Beispiel 1b:

In Analogie zu 1a wird E-2-[2-(3-Pyridyl)ethenyl]-benzaldehydethylenacetal erhalten

$^1$H-NMR (60 MHz, $CDCl_3$)$\delta$ = 4,2 (m, 4H, Methylen-H), 5,8 (s, 1H, Methin-H), 7,0-8,9 (m, 10H, olefinische und aromatische H).

Beispiel 1c:

In Analogie zu 1a wird E-3-[2-(3-Pyridyl)ethenyl]-benzaldehydethylenacetal erhalten:

$^1$H-NMR (60 MHz, $CDCl_3$):$\delta$ = 4,1 (m, 4H, Methylen-H), 5,9 (s, 1H, Methin-H), 7,1-8,9 (m, 10H, olefinische und aromatische H).

Beispiel 2a:

E-4-[2-(3-Pyridyl)ethyl]benzaldehyd

8.56 g (33.8 mmol) Acetal 1a wurden in 80 ml 1 n wäßriger Salzsäure gelöst und 3.5 h bei Raumtemp. gerührt. Anschließend goß man die Lösung vorsichtig unter Rühren in 100 ml gesättigte wäßrige Natriumhydrogencarbonatlösung. Um ausgefallenes Produkt in Lösung zu bringen, setzte man etwa 100 ml Toluol zu und rührte intensiv bei Raumtemp.. Man trennte die Phasen, sättigte die wäßrige mit Natriumchlorid und extrahierte zweimal mit Toluol. Trocknen der vereinigten organischen Phasen über Magnesiumsulfat, Abziehen des Lösungsmittels am Rotationsverdampfer und schließlich an der Ölpumpe ergab 6.96 g (33.3 mmol, 98 %) Aldehyd 2a als gelben Feststoff.

Schmp. 69-73 °C
IR (KBr): 1690 $cm^{-1}$ (s, Carbonyl)
$^1$H-NMR ($CDCl_3$, 60 MHz): $\delta$ = 7,0-8,8 (m, 10H, olefinische und aromatische H), 10,0 (s, 1H, -CHO)

Beispiel 2b:

In Analogie zu 2a wird E-2-[2-(3-Pyridyl)ethenyl]-benzaldehyd erhalten

NMR: ($CDCl_3$) $\delta$-Werte: 60 MHz-Spektrum
6.9-8.9 (m, 10H, aromat. + olefin. Protonen)
10.3 (s, 1H, CH=O)

Beispiel 2c:

In Analogie zu 2a wird E-3-[2-(3-Pyridyl)ethenyl]-benzaldehyd erhalten.
IR (KBr): 1690 $cm^{-1}$ (s, Carbonyl)
$^1$H-NMR (60 MHz, $CDCl_3$): $\delta$ = 6,9-8,8 (m, 10H, olefinische und aromatische H), 9,9 (s, 1H, -CHO)

Beispiel 3a:

4-[2-(3-Pyridyl)ethyl]benzaldehydethylenacetal

10 g (39.5 mmol) E-4-[2-(3-Pyridyl)ethenyl]benzaldehyd-ethylenacetal (1a) wurden in 100 ml Ethanol gelöst und mit 1.6 g Palladium auf Kohle (10 %) versetzt und bei Raumtemperatur und Normaldruck hydriert. Abfiltrieren und Abziehen des Lösungsmittels im Vakuum ergab 8.6 g (33.7 mmol, 85 %) eines weißen Feststoffes.
Schmp.: 46-47 °C
$^1$HNMR (60 MHz, $CDCl_3$): $\delta$ = 2,8 (m, 4H, $-CH_2CH_2-$), 3,9 (m, 4H, Acetalmethylen-H), 5,6 (s, 1H, Methin-H), 7,0 - 8,2 (m, 8H, aromatische H)

Beispiel 3b:

In Analogie zu 3a wird 2-[2-(3-Pyridyl)ethyl]benzaldehyd-ethylenacetal erhalten.

$^1$H-NMR (60 MHz, $CDCl_3$): $\delta$ = 2,7 (m, 4H, $CH_2CH_2$), 3,8 (m, 4H, Acetalmethylen-H), 5,7 (s, 1H, Methin-H), 7,1-8,3 (m, 8H, aromatische H)

Beispiel 3c:

In Analogie zu 3a wird 3-[2-(3-Pyridyl)ethyl]benzaldehyd-ethylenacetal erhalten

$^1$H-NMR (60 MHz, $CDCl_3$): $\delta$ = 2,8 (m, 4H, $-CH_2CH_2-$), 3,9 (m, 4H, Acetalmethylen-H), 5,8 (s, 1H, Methin-H), 7,0-8,2 (m, 8H, aromatische H).

Beispiel 4a:

4-[2-(3-Pyridyl)ethyl]benzaldehyd

8.5 g (33.7 mmol) Acetal 3a wurden in 85 ml 1n Salzsäure gelöst und 3.5 h bei Raumtemp. gerührt. Die Lösung wurde mit 80 ml gesättigter Natriumhydrogencarbonatlösung neutralisiert und dreimal mit Toluol extrahiert. Trocknen der vereinigten Toluolphasen mit Magnesiumsulfat und Abziehen des Lösungsmittels im Vakuum ergab 6,1 g (28.9 mmol, 86 %) Aldehyd 4a als gelbliches Öl.
IR (Film): 1690 $cm^{-1}$ (s, Carbonyl)
$^1$H-NMR (60 MHz, $CDCl_3$): $\delta$ = 2,9 ("s", 4H, $-CH_2CH_2-$), 6,9-8,4 (m, 8H, aromatische H), 9,7 (s, 1H, -CHO).

Beispiel 4b:

In Analogie zu 4a wird 2-[2-(3-Pyridyl) ethyl]benzaldehyd erhalten.

IR (Film): 1690 $cm^{-1}$ (s, Carbonyl)
$^1$H-NMR (60 MHz, $CDCl_3$): $\delta$ = 2,8 ("s", 4H, $-CH_2CH_2-$), 7,0 - 8,3 (m, 8H, aromatische H), 9,8 (s, 1H, -CHO)

Beispiel 4c:

In Analogie zu 4a wird 3-[2-(3-Pyridyl)ethyl]- benzaldehyd erhalten.

IR (Film): 1690 $cm^{-1}$ (s,Carbonyl )
$^1$H-NMR (60 MHz, $CDCl_3$): $\delta$ = 2,9 (m, 4H, $-CH_2CH_2-$), 7,0 - 8,4 (m, 8H, aromatische H), 9,9 (s, 1H, -CHO)

Beispiel 5:

2-Oxo-3-pentyloxypropanphosphonsäuredimethylester

Unter Argonatmosphäre wurden in 200 ml trockenem Tetrahydrofuran 25,6 g (0.2 Mol) Methanphosphonsäuredimethylester vorgelegt. In zwei Tropftrichtern wurden jeweils 250 ml 1.6 molare hexanische Lithiumbutyllösung (0.4 Mol) und 100 ml einer Lösung von 32.04 g (0.2 Mol) 2-Pentyloxyessigsäuremethylester in Tetrahydrofuran vorgelegt. Bei -70 °C bis -65 °C wurden dann 125 ml der Lithiumbutyllösung und gleich danach 50 ml der Esterlösung zugetropft. Man rührte 1 h bei -70 °C und tropfte anschließend weitere 63 ml der Lithiumbutyllösung und 25 ml der Esterlösung bei -70 °C zu und ließ 1h bei dieser Temperatur rühren. Schließlich wurden die restlichen 62 ml der Lithiumbutyllösung und 25 ml der Esterlösung bei der gleichen Temp. zugetropft und 2h gerührt. Man ließ über Nacht in Trockeneis stehen.

Zur Aufarbeitung wurde die Lösung bei 0-5 °C mit ca 160 ml 2 n wäßriger Salzsäure auf pH 5 eingestellt und anschließend am Rotationsverdampfer das THF abgezogen. Der Rückstand wurde mit gesättigter wäßriger Natriumchloridlösung aufgenommen und viermal mit Essigester extrahiert. Trocknen der vereinigten Essigesterphasen über Magnesiumsulfat, Abziehen des Lösungsmittels im Vakuum und fraktionierte Destillation ergab 37,0 g (0.147 Mol, 73 %) Phosphonat als farblose Flüssigkeit, Sdp. 130 - 132 °C / 0.5 Torr.
IR (Film): 1730 $cm^{-1}$ (s, Carbonyl)
$^1H$-NMR (60 MHz, $CDCl_3$): $\delta$ = 0,8-1,8 (m, 9H, $-(CH_2)_3CH_3$), 3,16 (d, J=22 Hz, 2H, P-$CH_2$-), 3,47 (t, J=6 Hz, 2H, -$OCH_2$-), 3,75 (d, J=11 Hz, 6H, -$OCH_3$), 4,1 (s, 2H, -$CH_2$-)

In Analogie zu Beispiel 5 werden die Phosphonate der Formel III erhalten. Die als Ausgangsmaterial verwendeten Ester sind weitgehend literaturbekannt oder werden in Analogie zu bekannten Verfahren hergestellt.

Beispiel 6a:

E,E-4-Pentyloxy-1-[4-(2-(3-pyridyl)ethenyl)phenyl] but-1-en-3-on

1,57 g (6.21 mmol) 2-Oxo-3-pentyloxypropanphosphonsäuredimethylester, 1,0 g (6.54 mmol) DBU und 1,3 g (6.21 mmol) 4-[2-(3-Pyridyl)ethenyl]benzaldehyd (2a) wurden in 150 ml Dimethoxyethan 22 h bei Raumtemp. gerührt. Das Lösungsmittel wurde im Vakuum abgezogen und der Rückstand auf 200 g Kieselgel (Petrolether/Essigester = 1:1, Rf = 0,38) chromatographiert. Nach der Kristallisation aus Ether/n-Hexan erhielt man 1.4 g (4,17 mmol, 67 %) Enon 6a.

Schmp.: 78 - 80 °C
IR (KBr): 1700 $cm^{-1}$ (Carbonyl)
$^1$H-NMR (60 MHz, $CDCl_3$): $\delta$ = 0,6-2,0 (m, 9H, $(CH_2)_3CH_3$), 3,5 (t, 2H, $OCH_2$-), 4,25 (s, 2H, $OCCH_2O$), 6,90-8,75 (m, 12H, olefinische und aromatische H)

In Analogie zu Beispiel 6a werden weitere u.a. die in Tabelle 1 angeführten Beispiele 6b - 6t erhalten.

Tabelle 1

| Beispiel | 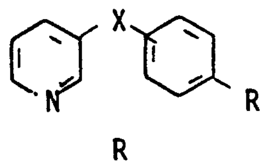 | charakteristische $^1$H-NMR-Signale ($CDCl_3$, 60 MHz), δ | Rf, Kieselgel |
|---|---|---|---|
| | X = -CH = CH-, E | | |
| 6b | 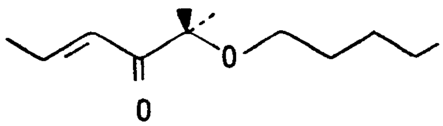 para | 0,6-2,0 (m, 15H, Methyl und $CH_2)_3CH_3$) | 0,5 Essigester |
| 6c | 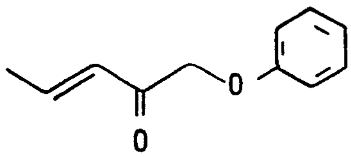 para | 4,8 (s, 2H, $-CH_2OPh$) | 0,4 Essigester |
| 6d | 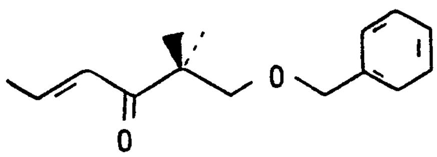 para | Fp. 80-82 °C 1.2(s,6H,$CH_3$); 3.5(s,2H,$C\underline{H}_2O$), 4.5(s,2H,$\underline{CH}_2OC_6H_5$)7.1-8.65(m, 17H,aromat. olefin.Protonen) | 0.38 $CH_2Cl_2$/MeOH = 20 : 1 |
| 6e | 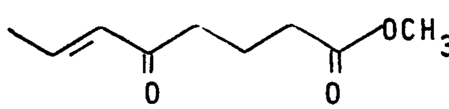 para | Fp.118-120 °C 1.9-2.9 (m,6H, $CH_2$), 3.7(s,3H, $COOCH_3$); 6.6-8.9 (m,12H,aromat. olefin. Protonen) | 0.59 $CH_2Cl_2$/MeOH = 10:1 |

| | | | |
|---|---|---|---|
| 6f | 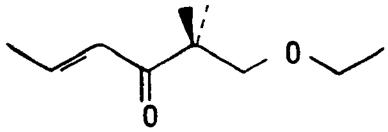 para | 1,17(t,3H,$-CH_2-CH_3$); 1,25(s,6H, $H_3CCCH_3$), 3,5 (q und t, 4H, Methylen-H) | 0.41 Essigester |
| 6g | 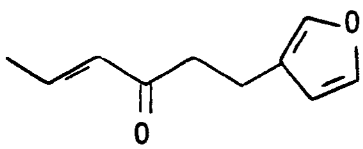 para | 3,o ("s", 4H, Methylen-H) | 0.35 EE/n-Hexan = 7:3 |
| 6h | 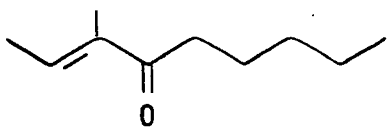 para | Fp 110-112 °C; 0.9 0.9(t,3H,$CH_3$) 1.1-1.9(m,6H,$CH_2$); 2.1 (d,3H,$CH_3$); 2.65-2.95 (m,2H,$CH_2CO$) 7.1-8.9 (m,11H, aromat., olefin. Protonen) | 0.37 $CH_2Cl_2$/MeOH = 20:1 |
| 6i | 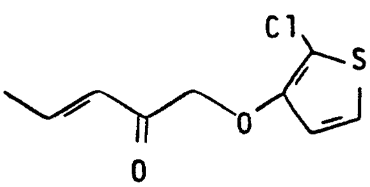 para | Fp. 113 °C 4.85 (s, 2H, $CH_2$ O) , 6.6-8.9 (m, 14H, aromat., olefin. Protonen) | 0.6 $CH_2Cl_2$/MeOH)=10:1 |
| 6j | 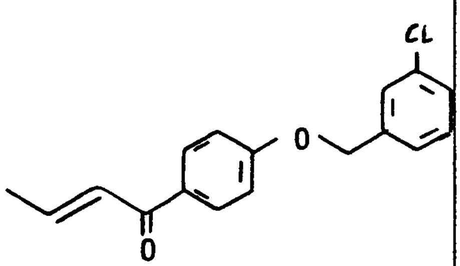 Para | 5.1(s,2H,$CH_2O$) 7.0-8.8 (m, 20H, aromat., olefin. Protonen) | 0.62 $CH_2Cl_2$/MeOH=10:1 |

| | | | |
|---|---|---|---|
| 6k | 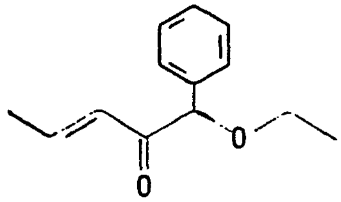<br>para | 1.4(t,3H,$CH_3$),<br>3.6(q,2H,$CH_2O$),<br>4.95(s,1H,CHO),<br>7.0-8.8(m,17H,<br>aromat., olefin.<br>Protonen | 0.45<br>Essigester/<br>MeOH<br>=8:2 |
| 6l | 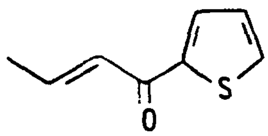<br>para | 6.5-8.8(m,15H,<br>aromat., olefin.<br>Protonen) | 0.43<br>Essigester/<br>MeOH<br>=8:2 |
| 6m | 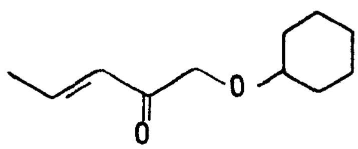<br>para | 1,0-2,2(m,10H,<br>Methylen-H),<br>3,33 (m, 1H,<br>Methin-H), 4,23<br>(s,2H,$-OCH_2-$) | 0.48<br>Essigester |
| 6n | 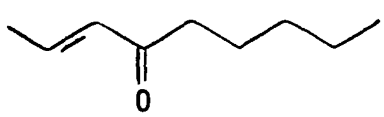<br>ortho | 0.9(t,3H,$CH_3$),<br>1.0-2.0(m,6H,$CH_2$),<br>2.6(t,2H,$CH_2CO$),<br>6.5-8.8(m,12H,<br>aromat. olefin.<br>Protonen) | Rf =<br>0.42<br>Cyclohexan/<br>Essigester=<br>=1:1 |
| 6o | 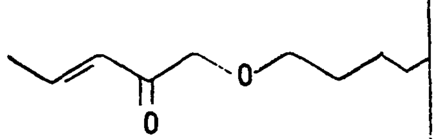<br>ortho | 0.8-2.1(m,9H,<br>Alkyl-H), 3.5<br>(t,2H,$-OCH_2-$)<br>4.2(s,2H,<br>$-OCCH_2O-$) | 0.35<br>EE/n-Hexan<br>= 2:1 |

| | | | |
|---|---|---|---|
| 6p | 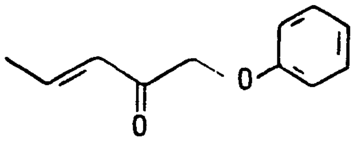 meta | 4,9(s,2H, -$\underline{CH}_2$OPh) | 0.45 Essigester |
| | $X = -CH_2-CH_2-$ | | |
| 6q | 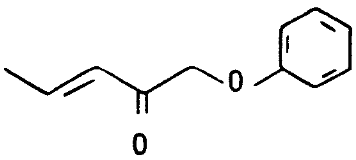 para | 2.96 ("s",4H, $-CH_2CH_2-$), 4,76(s,2H, $-CH_2OPh$) | 0.37 Essigester |
| 6r | 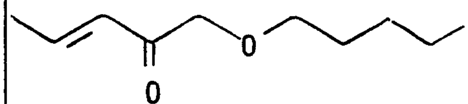 para | 0,6-2,0(m,9H, Alkyl-H), 2.96 ("s",4H,$-CH_2CH_2-$) 3.5(t,2H,$OCH_2-$), 4.23(s,2H,$OCCH_2O$) | 0.46 Essigester/ n-Hexan = 7:3 |
| 6s | 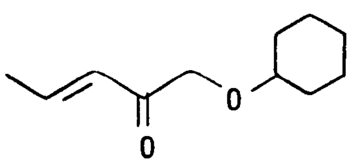 ortho | 0,9-2,2(m,10H, Methylen-H), 3.0("s",4H, $-CH_2CH_2-$), 3,3 (m,1H,Methin-H), 4,21 (s,2H, $-OCH_2-$) | 0.41 Essigester/ n-Hexan = 7:3 |
| 6t | 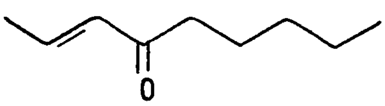 meta | 0,6-2,0(m,9H, $(CH_2)_3CH_3$),2,65 (t,2H,$-COCH_2-$) 3.0 ("s",4H, $-CH_2CH_2-$) | 0.38 Essigester/ Cyclohexan= =1:1 |

Beispiel 7a

E,E-4-Phenoxy-1-[4-(2-(3-pyridyl)ethenyl)phenyl]-but-1-en-3-ol

0,3 g (7.93 mmol) Natriumboranat wurden in 20 ml Methanol und 4 ml Tetrahydrofuran bei -5 °C vorgelegt. Bei -5 °C wurden 0,8 g (2.51 mmol) Enon 6c in 10 ml Methanol und 4 ml Tetrahydrofuran zugetropft. Man ließ 60 min bei dieser Temperatur rühren, säuerte mit einer wäßrigen Zitronensäurelösung an und zog das Lösungsmittel im Vakuum weitgehend ab. Der Rückstand wurde zwischen gesättigter Natriumhydrogencarbonatlösung und Methylenchlorid verteilt. Die wäßrige Phase wurde zweimal mit Methylenchlorid extrahiert. Trocknen der organischen Phasen mit Magnesiumsulfat und Abziehen des Lösungsmittels im Vakuum ergab nach Chromatographie an Kieselgel ($CH_2Cl_2/CH_3OH$ = 9:1) 0.78 g (2,27 mmol, 90 %) Alkohol 7a.

Schmp.: 106-108 °C

IR (KBr): 3250 $cm^{-1}$ (s, OH)

$^1$H-NMR: (60 MHz, $CDCl_3$): $\delta$ = 2,83 (breit, 1H, OH), 4,08 (m, 2H, Methylen-H), 4,73 (m, 1H, Methin-H), 6,0-8,7 (m, 17H, olefinische und aromatische H)

In Analogie zu Beispiel 7a werden weitere u.a. die in Tabelle 2 angeführten Beispiel 7b-7h erhalten.

Tabelle 2



| Beispiel | 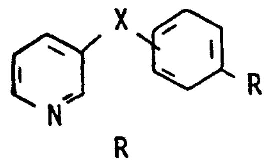 | charakteristische $^1$H-NMR-Signale ($CDCl_3$, 60 MHz), δ | Rf, Kieselgel |
|---|---|---|---|
| | X = -CH=CH-,E | | |
| 7b | 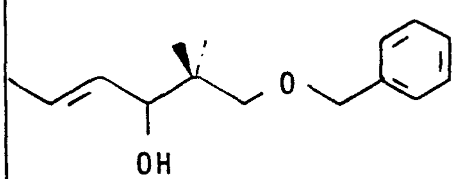<br>para | Fp. 114 °C<br>1.0(d,6H,$CH_3$),<br>3.2-3.6(m,2H,$CH_2O$),<br>4.55(s,2H,$CH_2OC_6H_5$),<br>6.0-6.8(m,2H,CH=CH),<br>7.1-8.9(m,13H,<br>aromat., olefin.<br>Protonen | 0.5<br>$CH_2Cl_2$/MeOH<br>= 20 : 1 |
| 7c | 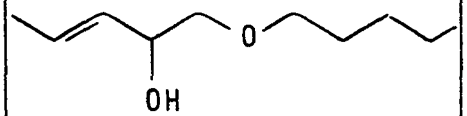<br>para | 0.65-2,0 (m,9H,<br>$(CH_2)_3CH_3$), 3.30-<br>3.81(m,4H,<br>$-CH_2OCH_2-$),4.31-<br>4.59 (m,1H,<br>Methin-H) | 0.33<br>Essigester/<br>Cyclohexan<br>= 2:1 |
| 7d | 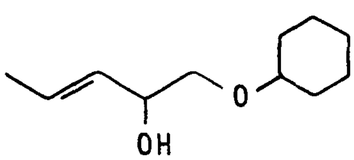<br>ortho | 1,01-2,32 (m,10H,<br>Methylen-H),<br>4,5(m,1H,Methin-<br>H) | 0.48<br>Essigester |
| 7e | 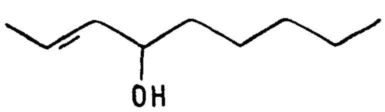<br>ortho | 0.9(t,3H,$CH_3$),<br>1.0-2.0(m,6H,$CH_2$),<br>4.1-4.5(m,1H,CHOH),<br>5.9-6.9(m,2H,CH=CH),<br>7.0-8.8(m,10H,<br>aromat., olefin.<br>Protonen) | 0.24<br>Cyclohexan/<br>Essigester<br>=1:1 |

| X = $-CH_2-CH_2-$ | | | |
|---|---|---|---|
| 7f | 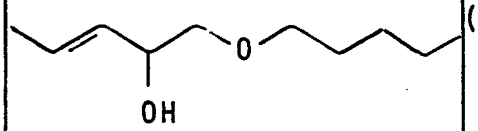 para | 0,66-1,83(m,9H, $(CH_2)_3CH_3$),2,90 ("s",4H,$-CH_2CH_2-$), 3,5(m,4H, $-CH_2OCH_2-$),4,5 (m,2H,Methin-H) | 0,34 Essigester/ n-Hexan = 7:3 |
| 7g | 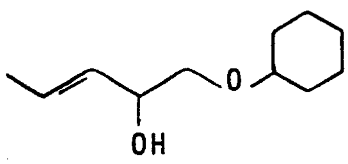 ortho | 1,0-2,3(m,10H, Methylen-H), 2,92("s",4H, $-CH_2CH_2-$), 3,3 (m,1H,Methin-H), 4,5(m,1H, Methin-H) | 0,32 Essigester/ n-Hexan= 7:3 |
| 7h | 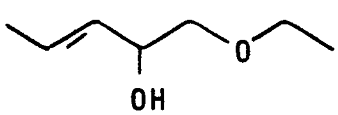 meta | 1,23(t,3H,$-CH_3$), 2,92("s",4H, $-CH_2CH_2-$), 3,16-4,0 (m,4H, $-CH_2OCH_2-$)4,5 (m,1H,Methin-H) | 0,38 Essigester |

Beispiel 8a

E,E-4-Phenoxy-3-(3-pyridylcarbonyloxy)-1-[4-(2-(3-pyridyl)ethenyl)phenyl]but-1-en-3-ol

440 mg (1,30 mmol) Alkohol 7a und 550 mg (3,1 mmol) 3-Pyridincarbonsäurechlorid-hydrochlorid wurden in 15 ml Pyridin und 15 ml Methylenchlorid über Nacht bei Raumtemp. gerührt und anschließend 8h unter Rückfluß erhitzt. Die Mischung wurde auf gesättigte Natriumhydrogencarbonatlösung gegossen. Es wurde dreimal mit Methylenchlorid extrahiert und die vereinigten Phasen mit Magnesiumsulfat getrocknet. Abziehen des Lösungsmittels im Vakuum und Chromatographie an Kieselgel (Essigester, Rf = 0,19) ergab 470 mg (1,04 mmol, 80 %) als gelbliches Öl.
IR (Film): 1720 $cm^{-1}$ (Estercarbonyl)
$^{1}H$-NMR (60 MHz, $CDCl_3$): $\delta$ = 4,4 (d, 2H, Methylen-H), 6,0-9,3 (m, 22H, Methin-H, olefinische und aromatische H)

In Analogie zu Beispiel 8a werden weitere u.a. die in Tabelle 3 angeführten Beispiele 8b - d erhalten.

Tabelle 3

| Beispiel | 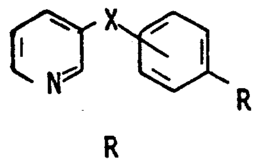 | charakteristische $^1$H-NMR-Signale ($CDCl_3$, 60 MHz), δ | Rf Kieselgel |
|---|---|---|---|
| | X = -CH=CH-,E | | |
| 8b | 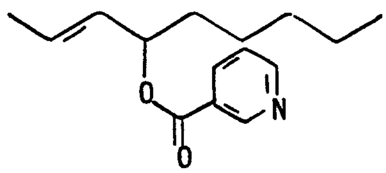<br>ortho | 0.9(t,3H,$CH_3$), 1.0-2.0(m,8H, $CH_2$), 4.9-5.4 (m, 1H, CHOCO-), 5.6-9.4(m,16H, aromat.,olefin. Protonen) | 0.17 Cyclohexan/ Essigester= 1:1 |
| 8c | 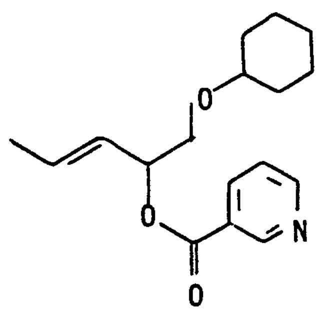<br>para | 1.0-2.3(m,10H, Methylen-H),3.77 (d,2H,-$CH_2$O-) 6.0-9.3 (m,17H, Methin-, olefin. und aromatische H) | 0.20 Essigester |
| | | | |

| | | | |
|---|---|---|---|
| | X = $-CH_2-CH_2-$ | | |
| 8d | 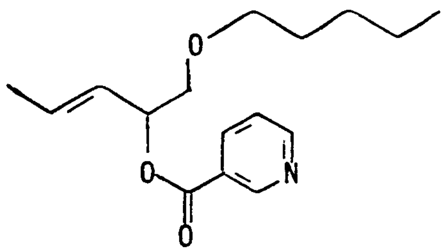 meta | 0.7-1.8(m,9H, $(CH_2)_3CH_3$),3.0 ("s",4H,$-CH_2CH_2-$), 3.3-3.8(m,4H, $-CH_2OCH_2-$)6.0-9.4 (m,17H, Methin-,olefinische und aromatische H) | 0.22 Essigester |
| 8e | 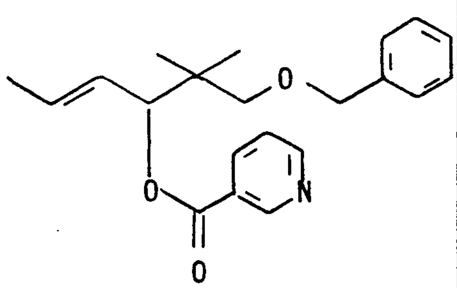 | 1.1(d,6H,$CH_3$), 3.3(s,2H,$CH_2O$), 4.45(s,2H, $CH_2OC_6H_5$); 5.7(d,1H,CHOCO-), 6.1-9.4(m,21H, aromat., olefin. Protonen) | 0.32 $CH_2Cl_2$/MeOH = 20:1 |

Beispiel 9a

E,E-4-Pentyloxy-1-[4-(2-(3-pyridyl)ethenyl)phenyl]-but-1-en-3-yl-benzylether

70 mg (1,6 mmol) 55 proz. NaH-Dispersion wurden in 3 ml trockenem Dimethylformamid vorgelegt. Bei Raumtemperatur gab man 375 mg (1,1 mmol) Alkohol 7c in 3 ml DMF zu und anschließend 152 mg (1,2 mmol) Benzylchlorid in wenig DMF. Man ließ 18 h bei Raumtemp. rühren, versetzte mit Wasser und extrahierte dreimal mit Toluol. Trocknen der vereinigten Toluolphasen über Magnesiumsulfat, Abziehen des Lösungsmittel im Vakuum und Chromatographie auf Kieselgel (Petrolether/Essigester = 2:1) ergab 232 mg (0,5 mmol, 45 %) Benzylether 9a als farblose Flüssigkeit.

$^1$H-NMR (60 MHz, $CDCl_3$): $\delta$ = 0.66-2.0 (m,9H,$-(CH_2)_3CH_3$), 3,31-3,63 (m, 4H, $-H_2COCH_2-$), 3,91-4,0 (m, 1H, Methin-H), 4,60 (d, 2H, $-OCH_2Ph$), 6,91-8,80 (m, 17H, olefinische und aromatische H).

In Analogie zu Beispiel 9a wurden weitere u.a. die in Tab. 4 aufgeführten Beispiele 9b - 9f erhalten.

Tabelle 4



| Beispiel | 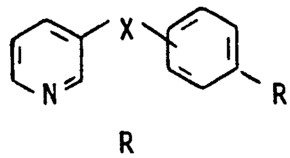 | charakteristische $^1H$-NMR-Signale, $\delta$ | Rf Kieselgel |
| --- | --- | --- | --- |
| | X = -CH=CH-, E | | |
| 9b | 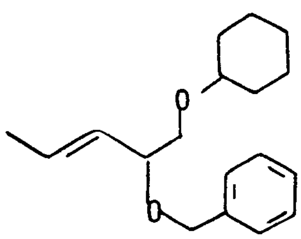 para | 1,0-2,1(m,10H, Cyclohexanmethylen-H), 3,4 (m,1H,Methin-H), 3,6(d,2H,$OCH_2$-), 4,2(m,1H,Methin-H),4,6(d,2H,Ph-$CH_2O$-) | 0.48 Essigester/ n-Hexan = 7:3 |
| 9c | 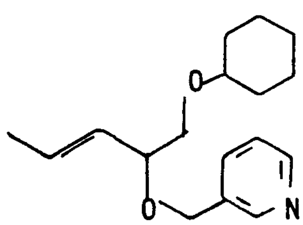 ortho | 1,0-2,1(m,10H, Cyclohexanmethylen-H),3,4 (m,1H,Methin-H), 3,5(d,2H,$OCH_2$-), 4,3(m,1H,Methin-H), 4,6("s", 2H,Ph-$CH_2O$), | 0.20 Essigester/ n-Hexan = 7:3 |

| X = $-CH_2-CH_2-$ | | | |
|---|---|---|---|
| 9d | 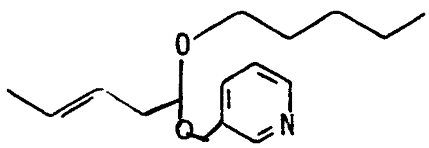 para | 0,7-1,9(m,9H, $-(CH_2)_3CH_3$),2,9 ("s",4H,$-CH_2CH_2$), 3,3-3,7(m,4H, $-CH_2OCH_2-$),4,0-4,7(m,1H,Methin-H), 4,6("s",2H, $-OCH_2$Pyr) | 0,19 Essigester/ n-Hexan = 7:3 |
| 9e | 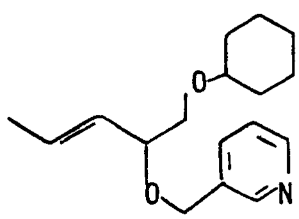 ortho | 1,0-2,0(m,10H, Cyclohexanmethylen-H), 3,4 (m,1H,Methin-H), 2,9("s",4H, $-CH_2CH_2-$), 3,6 (d,2H,$OCH_2-$), 4,3(m,1H,Methin-H), 4,6("s",2H, Pyr$CH_2O-$) | 0,20 Essigester/ n-Hexan = 7:3 |
| 9f | 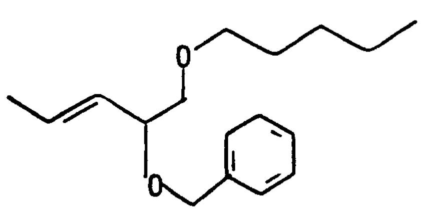 meta | 0,66-2,0(m,9H, $-(CH_2)_3CH_3$), 3,3-3,6 (m,4H, $-CH_2OCH_2-$), 3,9-4,4(m,1H, Methin-H), 4,6 (d,2H,$-OCH_2$Ph ) | 0,51 Essigester/ Cyclohexan = 3:1 |

0173172
HOE 84/F 193

Patentansprüche:

1. Verbindungen der Formel I

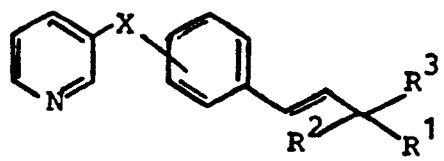

I

o, m, p

worin bedeuten:

X eine Vinylengruppe oder Ethylengruppe, $R^1$ und $R^2$ zusammen die Carbonylgruppe, oder $R^1$ Wasserstoff und $R^2$ der Rest $-OR^4$, worin $R^4$ Wasserstoff darstellt oder

a) einen verzweigten oder unverzweigtes aliphatischen Acylrest mit bis zu 10 C-Atomen,

b) den Benzylcarbonyl oder Benzoylrest, wobei der Phenylrest unsubstituiert oder 1- bis 3-fach substituiert ist mit Halogen oder $C_1$-$C_4$-Alkyl

c) den Rest 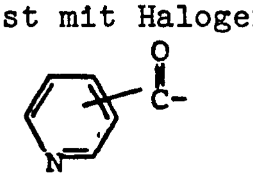

d) verzweigtes oder unverzweigtes Alkyl mit 1-10 C-Atomen,

e) den Benzylrest, wobei der Phenylkern unsubstituiert oder einfach substituiert ist mit Halogen oder $C_1$-$C_4$-Alkyl, oder

f) den Rest 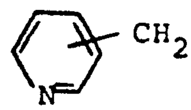 ,

$R^3$ einen Phenylrest, der im Kern 1- bis 3-fach substituiert sein kann mit Halogen, Trifluormethyl, und/oder Alkyl oder Alkoxy mit 1-6 C-Atomen, oder einen cycloaliphatischen Rest mit 3-8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3-8 Kohlenstoffatomen, wobei die aliphatischen Reste ihrerseits substituiert sein können mit

a) einem geradkettigen oder verzweigten Alkoxyrest mit bis zu 10 Kohlenstoffatomen oder einem Cycloalkoxyrest mit bis zu 6 Kohlenstoffatomen oder einem geradkettigen oder verzweigten Alkenyloxy- oder Alkinyloxyrest mit 3 bis 6 Kohlenstoffatomen,

b) Halogen, Cycloalkyl mit 3-7 C-Atomen, einem unsubstituierten Phenyl-, α- oder ß-Thienyl oder α- oder ß-Furylrest oder einem Phenyl-, Thienyl- oder Furylrest, welche ihrerseits im Kern 1- bis 3-fach substituiert sind mit Halogen, Trifluormethyl und/oder Alkyl oder Alkoxy mit 1-6 C-Atomen,

c) einem unsubstituierten Phenoxy-, α- oder ß-Thienyloxy-oder Cycloalkoxyrest mit 3-7 Kohlenstoffatomen oder einem der genannten Reste, welche seinerseits im Kern 1- bis 3-fach substituiert ist mit Halogen, Trifluormethyl und/oder Alkyl oder Alkoxy mit je 1-6 C-Atomen,

d) einem Heteroarylrest.

2. Verbindungen der Formel I gemäß Anspruch 1, worin bedeuten

$R^1$ und $R^2$ zusammen die Carbonylgruppe, oder $R^1$ Wasserstoff und $R^2$ den Rest $-OR^4$, worin $R^4$ Wasserstoff darstellt oder

a) verzweigtes oder unverzweigtes Alkanoyl mit bis zu 6 C-Atomen,

b) den Benzylcarbonyl- oder Benzoylrest, wobei der Phenylrest unsubstituiert oder einfach substituiert ist mit Fluor, Chlor der Methyl,

c) den Rest 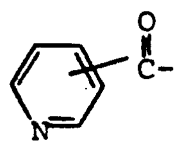 ,

d) verzweigtes oder unverzweigtes Alkyl mit 1-6 C-Atomen,

e) den Benzylrest, wobei der Phenylkern unsubstituiert oder einfach substituiert ist mit Chlor, Fluor oder Methyl, oder

f) den Rest 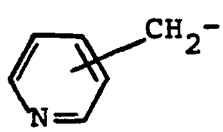 ,

$R^3$ einen cycloaliphatischen Rest mit 3-8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3 bis 8 Kohlenstoffatomen, wobei die aliphatischen Reste ihrerseits substituiert sein können mit

a) einem geradkettigen oder verzweigten Alkoxyrest mit bis zu 6 Kohlenstoffatomen oder einem Cycloalkoxyrest mit bis zu 6 Kohlenstoffatomen oder einem geradkettigen oder verzweigten Alkenyloxy- oder Alkinyloxyrest mit 3 bis 6 Kohlenstoffatomen,

b) Cycloalkyl mit 3-7 C-Atomen, einem unsubstituierten Phenyl-, α- oder ß-Thienyl oder α- oder ß-Furylrest oder einem Phenyl-, Thienyl- oder Furylrest, welche ihrerseits im Kern 1- bis 3-fach substituiert sind mit Halogen, Trifluormethyl und/oder Alkyl oder Alkoxy mit 1-6 C-Atomen,

c) einem unsubstituierten Phenoxy-, α- oder ß-Thienyloxy- oder Cycloalkoxyrest mit 3-7 Kohlenstoffatomen oder einem der genannten Reste, welche seinerseits im Kern 1- bis 3-fach substituiert ist mit Halogen, Trifluormethyl und/oder Alkyl oder Alkoxy mit je 1-4 C-Atomen,

d) einem Heteroarylrest.

3. Verbindungen der Formel II gemäß Anspruch 1, worin bedeuten

$R^1$ und $R^2$ zusammen die Carbonylgruppe, oder $R^1$ Wasserstoff und $R^2$ den Rest $-OR^4$ worin $R^4$ Wasserstoff, Benzyl, Alkyl mit 1-6 C-Atomen oder den Rest 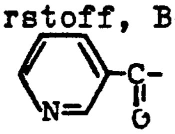

oder 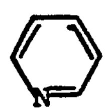 $-CH_2$-darstellt,

$R^3$ einen cycloaliphatischen Rest mit 3-8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3 bis 8 Kohlenstoffatomen, wobei die aliphatischen Reste ihrerseits substituiert sein können mit

a) einem geradkettigen oder verzweigten Alkoxyrest mit bis zu 10 Kohlenstoffatomen

b) Cycloalkyl mit 3 - 7 C-Atomen, einem unsubstituierten Phenyl-, α- oder β-Thienyl oder α- oder β-Furylrest oder einen Phenyl-, Thienyl- oder Furylrest, welche ihrerseits im Kern 1- bis 3-fach substituiert sind mit Halogen, Trifluormethyl und/oder Alkyl oder Alkoxy mit 1 - 6 C-Atomen,

c) einem unsubstituierten Phenoxy-, α- oder β-Thienyl-

oxy- oder Cycloalkoxyrest mit 3 - 7 Kohlenstoffatomen

d) einem 1-Imidazolylrest.

4. Verfahren zur Herstellung von Verbindungen der Formel I dadurch gekennzeichnet, daß man

a) die entsprechenden ortho-, meta- und parasubstituierten Benzaldehyde der Formel II

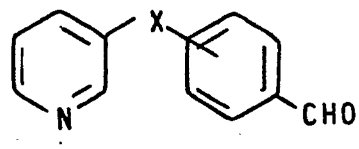

II

o, m, p,

nach Horner-Emmons-Wittig mit einem Phosphonsäureester der allgemeinen Formel III

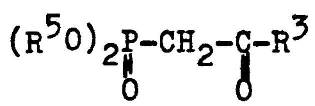

III

wobei $R^3$ die in der allgemeinen Formel I angegebene Bedeutung besitzt und $R^5$ $C_1$-$C_4$-Alkyl bedeutet, zu Verbindungen der allgemeinen Formel IV umsetzt,

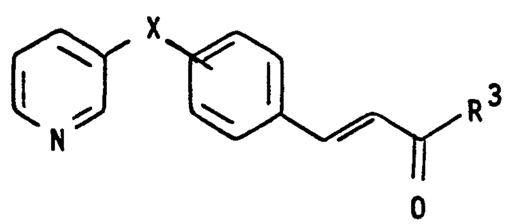

IV

o,m,p

wobei $R^3$ die zur Formel I angegebene Bedeutung hat,

b) gegebenenfalls die Enone der allgemeinen Formel IV mit einem Reduktionsmittel zu den Alkoholen der allgemeinen Formel V reduziert,

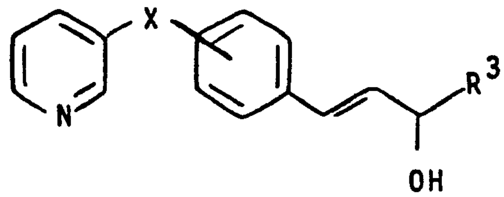

V

o,m,p

worin $R^3$ die zur Formel I gegebene Bedeutung hat,

c) gegebenenfalls die Alkohole der Formel V in Form ihrer Racemate oder als reine Enantiomere mit einem reaktionsfähigen Derivat einer Carbonsäure zu den Estern der allgemeinen Formel VI umsetzt,

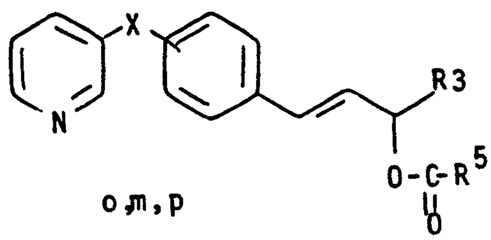

VI

o,m,p

worin $R^3$ die zur Formel I gegebene Bedeutung hat und $R^4$ einen verzweigten oder unverzweigtenaliphatischen Acylrest mit bis zu 10 C-Atomen, oder den Benzylcarbonyl oder Benzoylrest, worin der Phenylkern 1- bis 3-fach mit Halogen oder $C_1$-$C_4$-Alkyl substituiert sein kann, oder den Rest 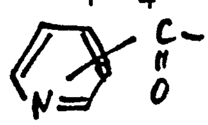 darstellt,

d) gegebenenfalls die Alkohole der Formel V in Form ihrer Racemate oder als reine Enantiomere mit einem entsprechenden Halogenid, Tosylat oder Mesylat zu den Ethern der allgemeinen Formel VII umsetzt,

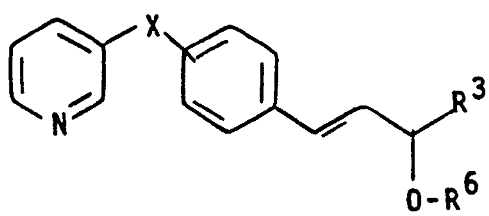

VII

o,m,p

worin $R^4$ $C_1$-$C_{10}$-Alkyl, Benzyl, worin der Phenylkern 1- bis 3-fach mit Halogen oder $C_1$-$C_6$-Alkyl substituiet sein kann, oder den Rest 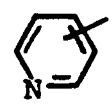 $CH_2$- darstellt.

5. Arzneimittel gekennzeichnet durch den Gehalt an einer Verbindung der Formel I gemäß Anspruch 1 in Mischung mit einem pharmazeutisch üblichen Träger und/oder Stabilisator.

6. Verwendung einer Verbindung der Formel I oder eines pharmazeutisch unbedenklichen Säureadditionssalzes zur Herstellung von Arzneimitteln zur Behandlung von Krankheiten mit erhöhter Thrombozytenaggregationsneigung.

Patentansprüche Österreich:

1. Verfahren zur Herstellung von Verbindungen der Formel I

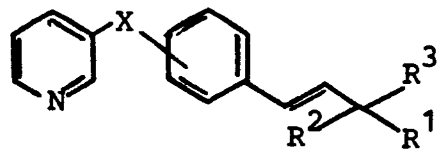

I

o, m, p

worin bedeuten:

$R^1$ und $R^2$ zusammen die Carbonylgruppe, oder $R^1$ Wasserstoff und $R^2$ der Rest $-OR^4$, worin $R^4$ Wasserstoff darstellt oder

a) einen verzweigten oder unverzweigtes aliphatischen Acylrest mit bis zu 10 C-Atomen,

b) den Benzylcarbonyl oder Benzoylrest, wobei der Phenylrest unsubstituiert oder 1- bis 3-fach substituiert ist mit Halogen oder $C_1$-$C_4$-Alkyl

c) den Rest 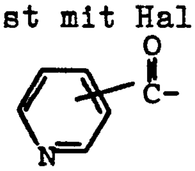

d) verzweigtes oder unverzweigtes Alkyl mit 1-10 C-Atomen,

e) den Benzylrest, wobei der Phenylkern unsubstituiert oder einfach substituiert ist mit Halogen oder $C_1$-$C_4$-Alkyl, oder

f) den Rest 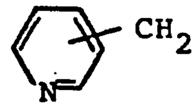 ,

$R^3$ einen Phenylrest, der im Kern 1- bis 3-fach substituiert sein kann mit Halogen, Trifluormethyl, und/oder Alkyl oder Alkoxy mit 1-4 C-Atomen, oder einen cycloaliphatischen Rest mit 3-8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3-8 Kohlenstoffatomen, wobei die aliphatischen Reste ihrerseits substituiert sein können mit

a) einem geradkettigen oder verzweigten Alkoxyrest mit bis zu 10 Kohlenstoffatomen oder einem Cycloalkoxyrest mit bis zu 6 Kohlenstoffatomen oder einem geradkettigen oder verzweigten Alkenyloxy- oder Alkinyloxyrest mit 3 bis 6 Kohlenstoffatomen,

b) Halogen, Cycloalkyl mit 3-7 C-Atomen, einem unsubstituierten Phenyl-, α- oder ß-Thienyl oder α- oder ß-Furylrest oder einem Phenyl-, Thienyl- oder Furylrest, welche ihrerseits im Kern 1- bis 3-fach substituiert sind mit Halogen, Trifluormethyl und/oder Alkyl oder Alkoxy mit 1-6 C-Atomen,

c) einem unsubstituierten Phenoxy-, α- oder ß-Thienyloxy-oder Cycloalkoxyrest mit 3-7 Kohlenstoffatomen oder einem der genannten Reste, welche seinerseits im Kern 1- bis 3-fach substituiert ist mit Halogen, Trifluormethyl und/oder Alkyl oder Alkoxy mit je 1-6 C-Atomen,

d) einem Heteroarylrest,

dadurch gekennzeichnet, daß man

a) die entsprechenden ortho-, meta- und parasubstituierten 1-Imidazolylmethylbenzaldehyde der Formel II

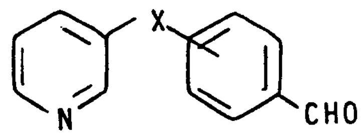

II

o, m, p,

nach Horner-Emmons-Wittig mit einem Phosphonsäureester der allgemeinen Formel III

$$(R^5O)_2\underset{\underset{O}{\|}}{P}-CH_2-\underset{\underset{O}{\|}}{C}-R^3 \qquad III$$

wobei $R^3$ die in der allgemeinen Formel I angegebene Bedeutung besitzt und $R^5$ $C_1$-$C_4$-Alkyl bedeutet, zu Verbindungen der allgemeinen Formel IV umsetzt,

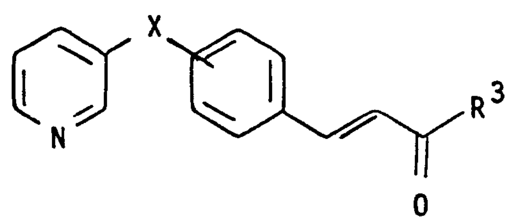

IV

o,m,p

wobei $R^3$ die zur Formel I angegebene Bedeutung hat,

b) gegebenenfalls die Enone der allgemeinen Formel IV mit einem Reduktionsmittel zu den Alkoholen der allgemeinen Formel V reduziert,

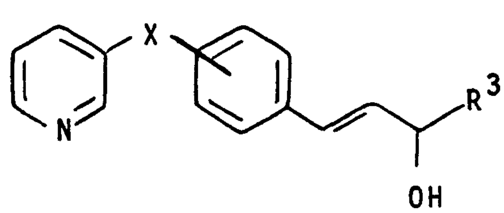

V

o,m,p

worin $R^3$ die zur Formel I gegebene Bedeutung hat,

c) gegebenenfalls die Alkohole der Formel V in Form ihrer Racemate oder als reine Enantiomere mit einem reaktionsfähigen Derivat einer Carbonsäure zu den Estern der allgemeinen Formel VI umsetzt,

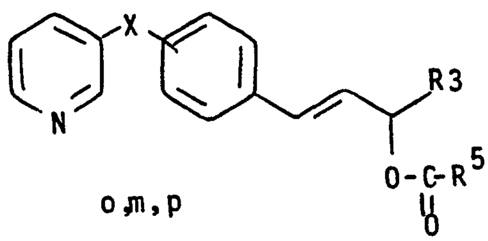

o,m,p

VI

worin $R^3$ die zur Formel I gegebene Bedeutung hat und $R^4$ einen verzweigten oder unverzweigtenaliphatischen Acylrest mit bis zu 10 C-Atomen, oder den Benzylcarbonyl oder Benzoylrest, worin der Phenylkern 1- bis 3-fach mit Halogen oder $C_1$-$C_4$-Alkyl substituiert sein kann, oder den Rest darstellt,

d) gegebenenfalls die Alkohole der Formel V in Form ihrer Racemate oder als reine Enantiomere mit einem entsprechenden Halogenid, Tosylat oder Mesylat zu den Ethern der allgemeinen Formel VII umsetzt,

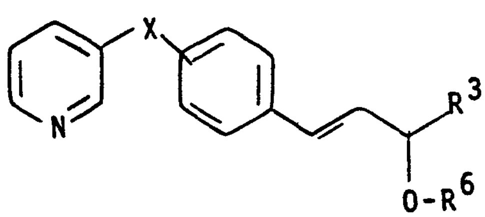

VII

o,m,p

worin $R^4$ $C_1$-$C_{10}$-Alkyl, Benzyl, worin der Phenylkern

1- bis 3-fach mit Halogen oder $C_1$-$C_4$-Alkyl substituiert sein kann, oder den Rest 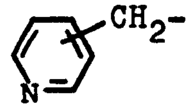 darstellt.

2. Verfahren gemäß Anspruch 1, dadurch gekennzeichnet, daß die Substituenten bedeuten:

$R^1$ und $R^2$ zusammen die Carbonylgruppe, oder $R^1$ Wasserstoff und $R^2$ der Rest $-OR^4$, worin $R^4$ Wasserstoff darstellt oder

a) verzweigtes oder unverzweigtes Alkanoyl mit bis zu 6 C-Atomen,

b) den Benzylcarbonyl oder Benzoylrest, wobei der Phenylrest unsubstituiert oder einfach substituiert ist mit Fluor, Chlor oder Methyl,

c) den Rest 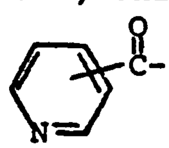

d) verzweigtes oder unverzweigtes Alkyl mit 1-6 C-Atomen,

e) den Benzylrest, wobei der Phenylkern unsubstituiert oder einfach substituiert ist mit Chlor, Fluor oder Methyl, oder

f) den Rest 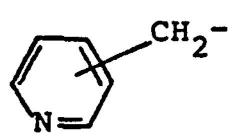 ,

$R^3$ einen cycloaliphatischen Rest mit 3-8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3 bis 8 Kohlenstoffatomen, wobei

die aliphatischen Reste ihrerseits substituiert sein können mit

a) einem geradkettigen oder verzweigten Alkoxyrest mit bis zu 6 Kohlenstoffatomen oder einem Cycloalkoxyrest mit bis zu 6 Kohlenstoffatomen oder einem geradkettigen oder verzweigten Alkenyloxy- oder Alkinyloxyrest mit 3 bis 6 Kohlenstoffatomen,

b) Cycloalkyl mit 3-7 C-Atomen, einem unsubstituierten Phenyl-, α- oder ß-Thienyl oder α- oder ß-Furylrest

c) einem unsubstituierten Phenoxy-, α- oder ß-Thienyloxy- oder Cycloalkoxyrest mit 3-7 Kohlenstoffatomen oder einem der genannten Reste, welche seinerseits im Kern 1- bis 3-fach substituiert ist mit Halogen, Trifluormethyl und/oder Alkyl oder Alkoxy mit je 1-4 C-Atomen,

d) einem Heteroarylrest.

3. Verfahren gemäß Anspruch 1, dadurch gekennzeichnet, daß die Substituenten bedeuten:

$R^1$ und $R^2$ zusammen die Carbonylgruppe, oder $R^1$ Wasserstoff und $R^2$ den Rest $-OR^4$ worin $R^4$ Wasserstoff, Benzyl, Alkyl mit 1-6 C-Atomen oder den Rest 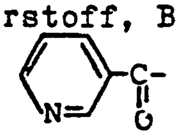

oder 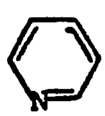 $-CH_2-$darstellt,

$R^3$ einen cycloaliphatischen Rest mit 3-8 Kohlenstoffatomen, einen geradkettigen oder verzweigten Alkylrest mit bis zu 8 Kohlenstoffatomen, einen geradkettigen oder verzweigten ungesättigten aliphatischen Kohlenwasserstoffrest mit 3 bis 8 Kohlenstoffatomen, wobei die aliphatischen Reste ihrerseits substituiert sein können mit

a) einem geradkettigen oder verzweigten Alkoxyrest mit bis zu 10 Kohlenstoffatomen

b) Cycloalkyl mit 3 - 7 C-Atomen, einem unsubstituierten Phenyl-, α- oder β-Thienyl oder α- oder β-Furylrest oder einen Phenyl-, Thienyl- oder Furylrest,

c) einem unsubstituierten Phenoxy-, α- oder β-Thienyloxy- oder Cycloalkoxyrest mit 3 - 7 Kohlenstoffatomen

d) einem 1-Imidazolylrest